# EUROPÄISCHE PATENTSCHRIFT

(11) **EP 1 245 560 B1**
(45) Veröffentlichungstag und Bekanntmachung des Hinweises auf die Patenterteilung: **04.06.2003**
(21) Anmeldenummer: 01105649.6
(22) Anmeldetag: 07.03.2001
(51) Int. Cl.: C07C 309/28, C08J 9/00, C08L 27/06

(54) **Zusammensetzung zur Herstellung von PVC-Schlagschäumen**
Composition for preparing frothed PVC foam
Composition pour la préparation de mousse battue de PVC

(43) Veröffentlichungstag der Anmeldung: 02.10.2002
(73) Patentinhaber: DR. TH. BÖHME KG CHEM. FABRIK GMBH & CO., D-82538 Geretsried (DE)
(72) Erfinder: Schönmann, Gertrud, Dr., 82166 Gräfelfing (DE); Ostermann, Jürgen, 82515 Wolfratshausen (DE); Ritschel, Susanne, 82538 Geretsried (DE); Hieb, Maria, 82538 geretsried (DE); Müller, Heike, 82538 Geretsried (DE)
(74) Vertreter: Störle, Christian, Dr.

(56) Entgegenhaltungen:
- DE-A- 2 848 672
- DE-C- 10 026 234
- PATENT ABSTRACTS OF JAPAN vol. 010, no. 321 (C-382), 31. Oktober 1986 (1986-10-31) & JP 61 130350 A (NIPPON ZEON CO LTD), 18. Juni 1986 (1986-06-18)

## Beschreibung

Die Erfindung bezieht sich auf die Verwendung einer speziellen Zusammensetzung zur Herstellung von PVC-Schlagschäumen.

PVC-Schaumstoffe haben eine Vielzahl unterschiedlicher Einsatzmöglichkeiten. Insbesondere werden sie zur Beschichtung von Materialien, z. B. als geschäumte Rückseiten von Fußbodenbelägen, verwendet. Beim gebräuchlichsten Verfahren zur Herstellung von PVC-Schaumstoffen wird Luft mechanisch in PVC-Plastisole eingeschlagen. Der dadurch erhaltene Schaum, der wie Schlagsahne aussieht, wird durch Erwärmen, z. B. auf 180°C, verfestigt (geliert). Dabei ist es erwünscht, daß die Dichte des Schaumes 0,6 g/cm³ bis 0,4 g/cm³ beträgt.

Zur Herstellung der PVC-Schlagschäume werden den PVC-Plastisolen Zusammensetzungen zugesetzt, die beispielsweise Calciumdodecylbenzolsulfonat enthalten, das als Schaumstandmittel wirkt. Gegebenenfalls wird es zusammen mit anderen Verbindungen, beispielsweise Fettsäureester, dem PVC-Plastisol zugegeben. Um die gewünschten Eigenschaften zu erreichen, müssen dem Plastisol große Mengen solcher Zusammensetzungen zugesetzt werden. Solche großen Mengen weisen aber den Nachteil auf, daß sie nur sehr schwer und umständlich handhabbar sind.

Aus der DE 28 48 672 A2 ist ein Verfahren zur Herstellung von Calciumsalzen von Alkylbenzolsulfonsäuren bekannt. Diese werden in Form ihrer alkoholischen Lösung als Komponenten für Mischemulgatoren, z.B. bei Pflanzenschutzformierungen, verwendet.

Der vorliegenden Erfindung liegt somit die Aufgabe zugrunde, eine Zusammensetzung für die Herstellung von PVC-Schlagschäumen ohne die Nachteile des Standes der Technik zu verwenden.

Erfindungsgemäß wird dies durch Verwendung einer Zusammensetzung zur Herstellung von PVC-Schlagschäumen erreicht, die sich dadurch auszeichnet, daß sie folgende Komponenten umfaßt:
(a) mehr als 35 Gew.-% bis 90 Gew.-% Erdalkalisalz einer Alkylbenzolsulfonsäure und
(b) 10 Gew.-% bis 65 Gew.-% ein- und/oder mehrwertiger Alkohol

Dabei beziehen sich die Mengenangaben auf die Zusammensetzung.

Es wurde nun überraschenderweise gefunden, daß diese Zusammensetzungen bestens zur Herstellung von PVC-Schlagschäumen geeignet sind. Dabei kann die Zusammensetzung in geringen Mengen dem PVC-Plastisol zugegeben werden, wobei PVC-Schlagschäume und PVC-Schaumstoffe mit hervorragenden Eigenschaften erhalten werden. Da im Vergleich zu bekannten Zusammensetzungen wesentlich geringere Mengen der erfindungsgemäß verwendeten Zusammensetzung eingesetzt werden müssen, um PVC-Schlagschäume und PVC-Schaumstoffe vergleichbarer Qualität zu erhalten, ist die erfindungsgemäß verwendete Zusammensetzung auch viel leichter handhabbar. Ferner ist die Herstellung geringer Mengen im Vergleich zur Herstellung großer Mengen wesentlich einfacher. Die erfindungsgemäße Verwendung vorstehender Zusammensetzung weist also ganz überraschende und vorteilhafte Eigenschaften auf.

Wie bereits vorstehend erwähnt wurde, wirkt sich die oben beschriebene Zusammensetzung vorteilhaft auf die Dichte der PVC-Schlagschäume aus. Die Dichte von PVC-Plastisolen beträgt üblicherweise etwa 1,3 g/cm³. Bei erfindungsgemäßer Verwendung der Zusammensetzung kann in einfacher Weise die gewünschte niedrige Dichte der Schlagschäume von z. B. 0,6 g/cm³ bis 0,4 g/cm³ erhalten werden. Des weiteren wirkt die erfindungsgemäß verwendete Zusammensetzung hervorragend als Schaumstandmittel; die PVC-Plastisole können somit in einfacher Weise zu mechanischen Schlagschäumen verarbeitet werden, die nicht zusammenfallen und gut weiterverarbeitet werden können.

Ferner werden durch die erfindungsgemäße Verwendung der Zusammensetzung in besonders günstiger Weise PVC-Schlagschäume mit besonders guten Eigenschaften erhalten, wie beispielsweise eine gute Streichfähigkeit, eine hervorragende Fließfähigkeit und ein sehr feinzelliger Schaum.

Als Komponente (a) liegt in der erfindungsgemäß verwendeten Zusammensetzung ein Erdalkalisalz einer Alkylbenzofsulfonsäure in einer Menge von 35 Gew.-% bis 90 Gew.-%, bezogen auf die Zusammensetzung, vor.

Vorzugsweise weist die Zusammensetzung 38 Gew.-% bis 65 Gew.-%, insbesondere 40 Gew.-% bis 60 Gew.-%, des Erdalkalisalzes einer Alkylbenzolsulfonsäure auf. Mit diesen Mengen können die Vorteile der erfindungsgemäßen Verwendung in besonders günstiger Weise erreicht werden. Insbesondere werden dünnflüssige Zusammensetzungen erhalten.

Die Alkylgruppe der Alkylbenzolsulfonsäure kann in o-, m- oder p-Position zur SO₃H-Gruppe der Benzolsulfonsäure stehen, wobei sie mit einem ihrer endständigen oder einem ihrer mittleren C-Atome an den Benzolring gebunden sein kann. Die Alkylgruppe kann linear oder verzweigt sein; sie kann 8 bis 16, insbesondere 12, C-Atome aufweisen. Das Erdalkali-lon kann Ca²⁺ sein. Besonders bevorzugt wird als Erdalkalisalz einer Alkylbenzolsulfonsäure Calciumdodecylbenzolsulfonat eingesetzt, da es ein besonders gutes Schaumstandmittel ist.

Die erfindungsgemäß verwendete Zusammensetzung kann ein oder mehrere, voneinander verschiedene Erdalkalisalze einer Alkylbenzolsulfonsäure aufweisen.

In der erfindungsgemäß verwendeten Zusammensetzung liegt als Bestandteil (b) 10 Gew.-% bis 65 Gew.-%, besonders bevorzugt 15 Gew.-% bis 50 Gew.-%, ein- und/oder mehrwertiger Alkohol vor. Durch diese Mengen werden überraschenderweise besonders günstige Eigenschaften der erfindungsgemäß verwendeten Zusammensetzung sowie der PVC-Schlagschäume und der PVC-Schaumstoffe erreicht.

Bei ein- bzw. mehrwertigen Alkoholen im Sinn der vorliegenden Erfindung handelt es sich um Hydroxy-Derivate aliphatischer oder alicyclischer, gesättigter oder ungesättigter Kohlenwasserstoffen mit 1 bzw. mehreren Hydroxy-Gruppen. Vertreter der mehrwertigen Alkohole sind zwei- und dreiwertige Alkohole.

Die Kohlenwasserstoffe können insbesondere von Alkanen, Alkenen oder Alkinen abgeleitet sein. Sind sie von Alkenen abgeleitet, können sie 1 oder mehrere, beispielsweise 2, Doppelbindungen aufweisen. Bei Alkinen können 1 oder mehrere, z.B. 2, Dreifachbindungen vorliegen.

Die das Grundgerüst der Alkohole bildenden Kohlenwasserstoffe können linear oder verzweigt sein.

Die Kohlenwasserstoffe können unsubstituiert oder substituiert sein. Liegen Substituenten vor, so bedeutet dies, daß zusätzlich zu den in den Alkoholen vorliegenden OH-Gruppen Substituenten anwesend sind. Dabei weist der Ausdruck "Substituent" darauf hin, daß ein H-Atom des Kohlenwasserstoffs durch einen in der organischen Chemie üblichen Substituenten ersetzt ist. Beispiele solcher sind die Halogene, wie Fluor, Chlor und Brom, die Nitro- und die Amino-Gruppe. Es können auch mehrere Substituenten vorliegen, die unabhängig voneinander gewählt werden.

Die Kettenlänge der Kohlenwasserstoffe wird vorzugsweise so gewählt, daß der Alkohol flüssig ist. Bei den zweiwertigen Alkoholen können die Kohlenwaserstoffe eine Kettenlänge von 2 bis 20 C-Atome aufweisen. Sind die Alkohole flüssig, sind sie besonders leicht und gut zu handhaben. Ferner können sie mit dem Erdalkalisalz einer Alkylbenzolsulfonsäure besonders gut gemischt werden.

Bei den zweiwertigen Alkoholen können die beiden Hydroxy-Gruppen am selben C-Atom, an benachbarten C-Atomen oder, insbesondere wenn das Diol von einem linearen Kohlenwasserstoff abgeleitet ist, an endständigen C-Atomen gebunden sein.

Die erfindungsgemäß verwendete Zusammensetzung weist mindestens 1 ein- oder mehrwertigen Alkohol auf. Demzufolge kann die Zusammensetzung auch mehrere voneinander verschiedene Alkohole aufweisen, beispielsweise 1 oder mehrere voneinander verschiedene einwertige Alkohole oder 1 oder mehrere voneinander verschiedene mehrwertige Alkohole oder auch ein Gemisch von 1 oder mehreren einwertigen Alkoholen mit 1 oder mehreren mehrwertigen Alkoholen.

Beispiele der einwertigen Alkohole sind Methanol, Ethanol, n-Propanol, iso-Propanol, Butanole und iso-Tridecanol.

Vertreter der zweiwertigen Alkohole sind 1,4-Butandiol, 1,2-Propylenglykol, 1,3-Propandiol, 1,3-Butandiol und 1,6-Hexandiol. Auch Gemische von zwei oder mehreren dieser können für die erfindungsgemäß verwendete Zusammensetzung verwendet werden. Als besonders gut hat sich 1,4-Butandiol erwiesen, da es zu besonders günstigen Schaumdichten führt. Dieses kann, wie bereits oben ausgeführt, zusammen mit anderen ein- oder zweiwertigen Alkoholen in der erfindungsgemäß verwendeten Zusammensetzung vorliegen, insbesondere mit vorstehenden Diolen.

Ein Beispiel eines dreiwertigen Alkohols ist Glycerin.

Vorzugsweise liegt in der erfindungsgemäß verwendeten Zusammensetzung weiterhin Wasser vor. Die Menge des Wassers beträgt beispielsweise 1 Gew.-% bis 10 Gew.-%, insbesondere 1 Gew.-% bis 3 Gew.-%, bezogen auf die Zusammensetzung. Überraschenderweise wurde gefunden, daß damit ein besonders gleichförmiger PVC-Schaum erhalten werden kann, in dem kleine Blasen gleichmäßig verteilt sind.

Die erfindungsgemäß verwendete Zusammensetzung kann weiterhin mindestens einen Bestandteil der Gruppe Mineralöle, Fettsäure, Fettsäureester, Verbindung der Formel (1) 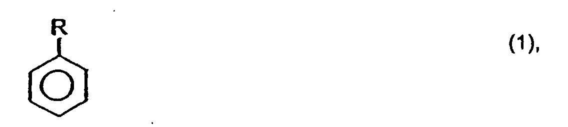 Verbindung der Formel (2) 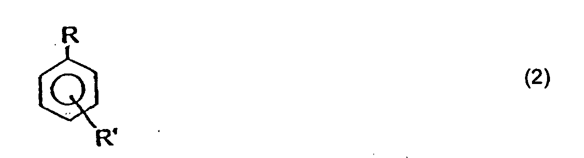 und Gemische von mindestens zwei dieser zusätzlichen Bestandteile enthalten, wobei R und R' unabhängig voneinander für einen substituierten oder unsubstituierten Alkyl-, Alkenyl-, Alkinyl-, Alkyloxy-, Alkenyloxy- oder Alkinyloxy-Rest mit 1 - 18 Kohlenstoff-Atomen oder O-[CH₂-CHR"-O-]ₘH stehen, wobei R" H oder CH₃ bedeutet und m 0 bis 13, insbesondere 1 - 4, ist.

Die Menge dieser weiteren Bestandteile kann so gewählt werden, daß sie den Rest der Zusammensetzung bilden.

Bei Mineralölen handelt es sich um eine Sammelbezeichnung für die aus mineralischen Rohstoffen, wie Erdöl, Braun- und Steinkohle, Holz, Torf, gewonnenen flüssigen Destillationsprodukten, die im wesentlichen aus Gemischen von gesättigten Kohlenwasserstoffen bestehen.

Die Fettsäuren können insbesondere gesättigte oder ungesättigte C₁₀-C₂₄-Fettsäuren sein, wie die Ölsäure.

Die Fettsäureester können Ester gesättigter oder ungesättigter C₂-C₂₄-Fettsäuren mit C₁-C₁₅-Alkoholen, wie 2-Ethyl-hexanol, sein.

Unter dem Ausdruck "Fettsäureester" werden auch Verbindungen verstanden, die durch Umsetzung von 1 Mol gesättigter oder ungesättigter Pflanzenfettsäure mit 12 bis 24 Kohlenstoff-Atomen, insbesondere 12 bis 18 Kohlenstoff-Atomen, mit 1 bis 2 Mol, z. B. 1,5 Mol, Alkylenoxid erhältlich sind. Als Alkylenoxid können Ethylenoxid und/oder Propylenoxid verwendet werden. Ein Beispiel der Pflanzenfettsäure ist Kokosfettsäure. Ein Vertreter des vorgenannten Fettsäureesters ist das Umsetzungsprodukt von 1 Mol Kokosfettsäure mit 1,5 Mol Propylenoxid.

Die in der erfindungsgemäß verwendeten Zusammensetzung eingesetzten Fettsäuren und Fettsäureester können flüssige Verbindungen sein. Sie können als Emulgatoren für die Bestandteile der erfindungsgemäßen Zusammensetzung wirken.

Liegt in den Verbindungen der Formeln (1) und (2) ein Alkenyl- oder Alkenyloxy-Rest vor, so kann dieser 1 oder mehrere, z. B. 2, Doppelbindungen aufweisen. Ist R und/oder R' ein Alkinyloder Alkinyloxy-Rest, so kann dieser 1 oder mehrere, z. B. 2, Dreifachbindungen besitzen.

Wie bereits vorstehend ausgeführt wurde, können die Alkyl-, Alkenyl-, Alkinyl-, Alkyloxy-, Alkenyloxy- oder Alkinyloxy-Reste substituiert sein, wobei sie einen oder mehrere Substituenten aufweisen können. Die Substituenten können solche sein, wie sie in der organischen Chemie verwendet werden. Beispiele der Substituenten sind die Hydroxy-Gruppe oder Halogene, wie F, Cl und Br. Liegen mehrere Substituenten vor, können diese gleich oder verschieden voneinander sein.

In den Verbindungen der Formeln (1) und (2) kann auch der Benzolring 1 oder mehrere Substituenten aufweisen, die unabhängig voneinander gewählt sind. Beispiele solcher sind die OH-Gruppe und Halogene, wie F, Cl und Br. Dabei können auch mehrere Substituenten an die Benzolringe gebunden sein, die gleich oder verschieden voneinander sein können.

Günstigerweise ist die Verbindung der Formel (1) ein Monoalkylbenzol. Liegt ein solches in der erfindungsgemäß verwendeten Zusammensetzung vor, werden besonders niedrige Dichten des Schlagschaums erhalten, und die unter Verwendung der Zusammensetzung hergestellten PVC-Schaumstoffe sind besonders thermostabil. Beim Gelieren des Schlagschaums werden keine Verfärbungen beobachtet; die erhaltenen PVC-Schaumstoffe bleiben weiß.

Der Alkylrest des Monoalkylbenzols kann linear oder verzweigt sein. Er kann über eines der terminalen C-Atome oder über eines der mittleren C-Atome an den Benzolring gebunden sein.

Besonders bevorzugt ist das Monoalkylbenzol ein C₆-C₁₈-Alkylbenzol. Dies bedeutet, daß ein Alkylrest mit 6 bis 18-Kohlenstoff-Atomen an den Benzolring gebunden sein kann. Vertreter eines solchen ist das Dodecylbenzol.

In der Verbindung gemäß Formel (2) kann der Rest R' in o-, m- oder p-Position zum Rest R stehen.

Ein Beispiel für die Verbindung der Formel (2) ist ein Dialkylbenzol oder ein Gemisch von Dialkylbenzolen. Als günstig hat es sich dabei erwiesen, daß das Dialkylbenzol ein lineares Dialkylbenzol oder ein Gemisch linearer Dialkylbenzole umfaßt. Die beiden Alkylketten können gleich oder verschieden lang sein, beispielsweise mit Kettenlängen von C₁₀-C₁₃.

Die Konstitution eines linearen Dialkylbenzols mit Alkylkettenlängen von C₁₀ bis C₁₃ kann durch folgende allgemeine Strukturformel veranschaulicht werden 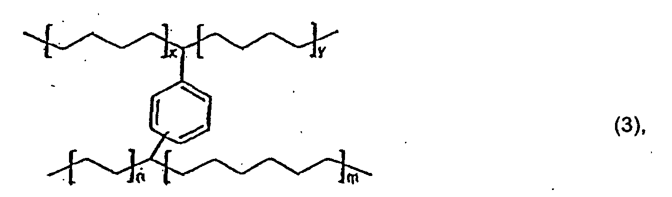 wobei x und y die Anzahl der C-Atome in der ersten Alkylkette,
m und n die Anzahl der C-Atome in der zweiten Alkylkette angeben,
x, y, m und n für eine Zahl von 0 bis 13 steht,
wobei die Gesamtlänge der Alkylketten 10 bis 13 Kohlenstoffatome beträgt.

Bei Verwendung eines Dialkylbenzols werden besonders niedrige Dichten des PVC-Schlagschaumes erhalten. Ferner sind die PVC-Schaumstoffe besonders thermostabil.

Die vorgenannten Monoalkyl- und Dialkylbenzole können durch Alkylierung von Benzol mit Chlorparaffinen unter Verwendung eines AlCl₃-Katalysators in üblicher Weise hergestellt werden.

Die Mengen des Erdalkalisalzes einer Alkylbenzolsulfonsäure (a), des ein- oder mehrwertigen Alkohols (b) sowie des Wassers und der gegebenenfalls vorliegenden zusätzlichen Bestandteile können so aufeinander abgestimmt sein, daß in der erfindungsgemäß verwendeten Zusammensetzung keine Phasentrennung auftritt und sie ferner flüssig ist. Dadurch ist die erfindungsgemäß verwendete Zusammensetzung besonders gut handhabbar.

Die erfindungsgemäß verwendete Zusammensetzung kann in einfacher Weise dadurch hergestellt werden, daß mindestens eine Alkylbenzolsulfonsäure, ein Erdalkalicarbonat, Wasser sowie mindestens ein ein- oder mehrwertiger Alkohol zusammengegeben werden. Die zusätzlichen Bestandteile können ebenfalls zugegeben werden. Die Reihenfolge der Zugabe kann beliebig gewählt werden. Nach der Zugabe und dem Mischen kann eventuell überschüssiges Wasser durch Destillation entfernt werden, um den Wassergehalt auf die vorstehend angegebenen Mengen einzustellen. Erdalkalicarbonate sind im allgemeinen schwer löslich. Der in der erfindungsgemäß verwendeten Zusammensetzung vorliegende ein- oder mehrwertige Alkohol kann überraschenderweise als Lösungsvermittler fungieren.

Die erfindungsgemäße Zusammensetzung eignet sich bestens zur Verwendung in einem Verfahren zur Herstellung von PVC-Schaumstoffen.

Der Ausdruck "PVC-Schaumstoffe" bezeichnet Werkstoffe auf der Basis von PVC, die über ihre ganze Masse verteilte offene Zellen und ein Rohdichte aufweisen können, die niedriger als die der PVC-Gerüstsubstanz sein kann. Der Schaumstoff kann grob- oder feinzellig sein. Er kann auch einen geringen Anteil an geschlossenen Zellen aufweisen, wobei es jedoch für die Luftdurchlässigkeit, insbesondere bei der Verwendung der Schaumstoffe als Rückseite von Fußbodenbelägen, günstig ist, wenn der weitaus überwiegende Teil, z. B. 90%, insbesondere 95%, der Zellen, offene Zellen sind. Diese Werkstoffe können jede beliebige Form aufweisen, abhängig von ihrem jeweiligen Verwendungszweck. Werden die Schaumstoffe als Rückseiten von Fußbodenbelägen verwendet, ist es günstig, wenn sie bahnförmig sind und eine Dicke von 1 mm bis 10 mm, insbesondere ca. 3 mm, aufweisen. Die Breite und Länge einer solchen Bahn ist größer als die Dicke.

Zur Herstellung von PVC-Schaumstoffen wird die erfindungsgemäß verwendete Zusammensetzung zunächst einem PVC-Plastisol in üblicher Weise zugegeben.

Bei einem PVC-Plastisol handelt es sich um eine Dispersion von PVC in hochsiedenden organischen Lösungsmitteln, die bei höherer Temperatur als Weichmacher fungieren. Beim Erwärmen der PVC-Plastisole diffundieren Lösungsmittel in die dispergierten Polymere, lagern sich zwischen deren Makromolekülen ein und bewirken dadurch ein Plastifizieren. Beim Abkühlen gelieren PVC-Plastisole zu flexiblen, formstabilen und abriebfesten Systemen.

Als Weichmacher werden vorzugsweise Phthalate, d. h. von Phthalsäure abgeleitete Verbindungen eingesetzt. Dabei kann ein Phthalat oder es können mehrere, z. B. zwei, voneinander verschiedene Phthalate verwendet werden. Zur Herstellung stabiler Schlagschäume ist es günstig, wenn mindestens 40% des Weichmachers schnell gelierend sind, wobei "schnell gelierend" ein auf diesem Gebiet üblicher Fachausdruck ist. Beispiele besonders geeigneter Phthalate sind Benzylbutylphthalat und Di-(2-ethylhexyl)phthalat, die besonders schnell gelierend sind. Als besonders günstig hat es sich erwiesen, wenn anteilsweise Benzylbutylphthalat im PVC-Plastisol vorhanden ist, da dadurch die Herstellung von feinzelligen Schäumen und Schaumstoffen besonders gut möglich ist. Die gesamte Weichmachermenge beträgt günstigerweise 40 bis 80 Gew.-Teile pro 100 Gew.-Teile PVC. Bei diesen Weichmachermengen haben die Plastisole eine für die Verschäumung besonders günstige Viskosität.

Der Ausdruck "PVC" umfaßt sowohl Polyvinylchloridhomopolymere als auch Polyvinylchloridcopolymere sowie deren Gemische. Dabei können im Plastisol mehrere, z. B. 2, voneinander verschiedene Polyvinylchloridhomopolymere vorliegen, die z. B. unterschiedliche Molekulargewichte aufweisen. Unter einem Polyvinylchloridcopolymer wird ein Copolymer aus Vinylchlorid und einem anderen, mit Vinylchlorid polymerisierbaren Monomor (Comonomer) verstanden, z. B. Vinylacetat, Vinylidenchlorid, Vinylether und Vinylester. Dabei kann der Comonomeranteil 5 bis 15 Gew.-% des Vinylchlorids betragen. Das Copolymer kann ein Blockoder ein Randomcopolymer sein. Im Plastisol können mehrere, z. B. 2, voneinander verschiedene Polyvinylchloridcopolymere vorliegen, die z. B. unterschiedliche Molekulargewichte und/oder unterschiedliche Comonomere aufweisen. Das Plastisol kann auch ein Gemisch von mehreren, z. B. 2, unterschiedlichen Polyvinylchloridhomopolymeren und von mehreren, z. B. 2, unterschiedlichen Polyvinylchloridcopolymeren enthalten.

Vorstehend beschriebene Polymere können z. B. durch Emulsionspolymerisation, Mikrosuspensionspolymerisation und Massepolymerisation hergestellt werden.

Zur Herstellung der PVC-Schaumstoffe wird, wie bereits vorstehend ausgeführt wurde, die vorstehend beschriebene Zusammensetzung zu einem PVC-Plastisol gegeben. Dieses Plastisol kann neben den vorstehend genannten Weichmachern noch weitere Verbindungen aufweisen, beispielsweise Stabilisatoren, wie ein Barium-Zink-Salz mittelkettiger Carbonsäuren, insbesondere in einer Menge von etwa 1 Gew.-Teil pro 100 Gew.-Teile Harz (PVC).

Die Menge der erfindungsgemäß verwendeten Zusammensetzung im PVC-Plastisol kann von 1 bis 2 Gew.-Teile pro 100 Gew.-Teile PVC betragen. Bei den aus dem Stand der Technik bekannten Zusammensetzungen zur Herstellung von PVC-Schäumen mußten größere Mengen als bei der erfindungsgemäß verwendeten Zusammensetzung eingesetzt werden, um die gleichen positiven Effekte bei den Schlagschäumen und den Schaumstoffen zu erzielen.

Nach Zugabe aller Bestandteile zum PVC-Plastisol wird dieses geschäumt, d. h. zu einem Schaum verarbeitet. Dieses Schäumen kann in üblicher Weise erfolgen, beispielsweise durch mechanisches Einschlagen von Luft, wobei ein an sich bekannter Schaummischer verwendet werden kann. Dies kann auch durch einen handelsüblichen Küchenmixer, z. B. von der Firma Kennwood, erfolgen.

Der Schaum wird dann in die gewünschte Form gebracht, z. B. durch Aufstreichen auf eine Unterlage, beispielsweise ein Trennpapier. Dieses Aufstreichen kann beispielsweise mittels eines Rakels erfolgen. Die Dicke der Schaumschicht kann 1 bis 10 mm, vorzugsweise 3 mm, betragen.

Danach wird der Schaum geliert, wodurch ein PVC-Schaumstoff erhalten wird. Das Gelieren kann durch Erwärmen, z. B. auf etwa 180°C, und anschließendem Abkühlen erfolgen. Die Dauer des Gelierens ist abhängig von der Form, in die der Schaum gebracht wurde. Wird der Schaum in Form einer Schicht mit einer Dicke von ca. 3 mm auf eine Unterlage aufgebracht, so beträgt die Gelierdauer etwa 5 Minuten. Nach dem Gelieren kann der Schaumstoff vom Trennpapier entfernt werden.

Durch die erfindungsgemäße Verwendung der vorstehend beschriebenen Zusammensetzung sind PVC-Schaumstoffe mit besonders guten Eigenschaften auf einfache, schnelle und kostengünstige Weise erhältlich.

Die nachfolgenden Beispiele erläutern die Erfindung.

### Beispiel 1: Herstellung erfindungsgemäß verwendeter Zusammensetzungen

Die in der nachfolgenden Tabelle 1 angegebenen Zusammensetzungen wurden hergestellt, indem Dodecylbenzolsulfonsäure, Calciumcarbonat, 1,2-Propylenglykol, 1,4-Butandiol und Wasser gemischt wurden, wobei die Dodecylbenzolsulfonsäure schrittweise zugegeben wurde, um eine zu heftige CO₂-Entwicklung zu vermeiden. Wasser kann durch Destillation entfernt werden.

**Tabelle 1:**

| Erfindungsgemäße Zusammensetzungen (Mengenangaben in Gew.-%) | | | | |
|---|---|---|---|---|
| Bestandteil | Z1 | Z2 | Z3 | Z4 |
| Calciumdodecylbenzolsulfonat | 38,7 | 56,3 | 62,5 | 40 |
| 1,2-Propylenglykol | 19,3 | 16,9 | 18,8 | |
| 1,4-Butandiol | 19,3 | 14,0 | 15,6 | 20 |
| Wasser | 2,7 | 2,8 | 3,1 | |
| freie Ölsäure | 20 | 10 | | 20 |
| Ölsäuremethylester | | | | 20 |

### Beispiel 2: Erfindungsgemäße Verwendung von Zusammensetzungen zur Herstellung von PVC-Schaumstoffen

Es wurden die in der nachfolgenden Tabelle 2 angegebenen Rezepturen unter Verwendung der Zusammensetzungen Z1, Z2, Z3 und Z4 des Beispiels 1 hergestellt.

**Tabelle 2:**

| Rezepturen für die Herstellung von PVC-Schaumstoffen | | | | |
|---|---|---|---|---|
| Rezeptur-Nr. (Gew.-Teile) | 1 | 2 | 3 | 4 |
| PVC (K-Wert 80) Vestolit® P 1415/K80 | 140 | 140 | 140 | 140 |
| PVC (K-Wert 65) Vinnolit® C65V | 60 | 60 | 60 | 60 |
| DOP | 50 | 50 | 50 | 50 |
| BBP | 50 | 50 | 50 | 50 |
| Stabilisator (Irgastab® BZ 505) | 2 | 2 | 2 | 2 |
| Z1 | 3,3 | | | |
| Z2 | | 2,2 | | |
| Z3 | | . | 2,0 | |
| Z4 | | | | 3,0 |
| Schaumdichte | 0,54 | 0,55 | 0,57 | 0,55 |

Vestolit® P 1415/K80 ist ein Mikrosuspensions-PVC der Vestolit GmbH mit einem K-Wert von 80; Vinnolit® C 65 V ist ein durch Suspensionspolymerisation hergestelltes Verschnittharz für PVC-Pasten mit einem K-Wert von 65 der Vinnolit GmbH; DOP ist die Abkürzung für Di-(2-ethylhexyl)phthalat; BBP ist die Abkürzung für Benzylbutylphthalat; Irgastab® BZ 505 ist ein handelsüblicher Barium-Zink-Stabilisator.

Die in Tabelle 2 aufgeführten Komponenten wurden zusammengegeben und zu einem Schaum geschlagen. Die dabei erreichten Schaumdichten sind ebenfalls in Tabelle 2 angegeben. Dabei handelt es sich um die gewünschten niedrigen Schaumdichten.

Von diesen Schäumen wurden Aufstriche auf Trennpapier in einer Dicke von 3 mm angefertigt und jede Probe jeweils während 5, 10, 15 und 20 Minuten bei 180 °C behandelt, wobei PVC-Schaumstoffe mit hervorragenden Eigenschaften erhalten werden.

## Patentansprüche

1. Verwendung einer Zusammensetzung, umfassend
(a) mehr als 35 Gew.-% bis 90 Gew.-% Erdalkalisalz einer Alkylbenzolsulfonsäure und
(b) 10 Gew.-% bis 65 Gew.-% ein- und/oder mehrwertiger Alkohol, zur Herstellung von PVC-Schlagschäumen.

2. Verwendung nach Anspruch 1, wobei 38 Gew.-% bis 65 Gew.-%, insbesondere 40 Gew.-% bis 60 Gew.-%, Erdalkalisalz einer Alkylbenzolsulfonsäure vorliegen.

3. Verwendung nach Anspruch 1 oder 2, wobei das Erdalkalisalz einer Alkylbenzolsulfonsäure Calciumdodecylbenzolsulfonat ist.

4. Verwendung nach einem der vorhergehenden Ansprüche, wobei 15 Gew.-% bis 50 Gew.-% ein- und/oder mehrwertiger Alkohol vorliegen.

5. Verwendung nach einem der vorhergehenden Ansprüche, wobei der Alkohol flüssig ist.

6. Verwendung nach einem der vorhergehenden Ansprüche, wobei der Alkohol 1,4-Butandiol, 1,2-Propylenglykol, 1,3-Propandiol, 1,3-Butandiol, 1,6-Hexandiol oder ein Gemisch von zwei oder mehreren davon ist.

7. Verwendung nach einem der vorhergehenden Ansprüche, wobei die Zusammensetzung weiterhin Wasser enthält.

8. Verwendung nach Anspruch 7, wobei 1 Gew.-% bis 10 Gew.-% Wasser vorliegen.

9. Verwendung nach einem der vorhergehenden Ansprüche, wobei die Zusammensetzung weiterhin mindestens einen Bestandteil der Gruppe Mineralöl, Fettsäure, Fettsäureester, Verbindung der Formel (1) 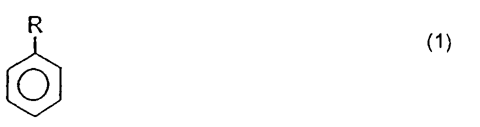 und Verbindung der Formel (2) 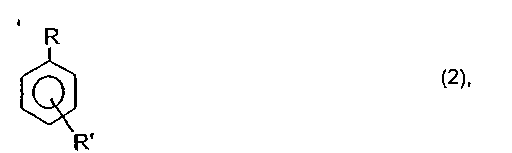 aufweist, wobei R und R' unabhängig voneinander für einen substituierten oder unsubstituierten Alkyl-, Alkenyl-, Alkinyl-, Alkyloxy-, Alkenyloxy- oder Alkinyloxy-Rest mit 1 - 18 Kohlenstoff-Atomen oder O-[CH₂-CHR"-O-]ₘH stehen, wobei R" H oder CH₃ bedeutet und m 0 bis 13, insbesondere 1 - 4, ist.

10. Verwendung nach Anspruch 9, wobei die Fettsäure eine C₁₀-C₂₄-Fettsäure ist

11. Verwendung nach Anspruch 9, wobei der Fettsäureester ein Ester einer C₂-C₂₄-Fettsäure mit einem C₁-C₁₅-Alkohol ist.

12. Verwendung nach Anspruch 9, wobei der Fettsäureester eine C₁₂-C₂₄-Pflanzenfettsäure mit 1 bis 2 von Alkylenoxid abgeleiteten Einheiten ist.

13. Verwendung nach einem der Ansprüche 9 bis 12, wobei das Mineralöl, die Fettsäure, der Fettsäureester, die Verbindung der Formel (1) und/oder die Verbindung der Formel (2) den Rest der Zusammensetzung bilden.

## Claims

1. Use of a composition comprising
(a) more than 35% by weight to 90% by weight of an alkaline earth metal salt of an alkylbenzenesulfonic acid and
(b) 10% by weight to 65% by weight of a monohydric alcohol and/or a polyhydric alcohol,
for the production of PVC frothing foams.

2. Use according to Claim 1 wherein 38% by weight to 65% by weight, especially 40% by weight to 60% by weight, of alkaline earth metal salt of an alkylbenzenesulfonic acid is present.

3. Use according to Claim 1 or 2 wherein the alkaline earth metal salt of an alkylbenzenesulfonic acid is calcium dodecylbenzenesulfonate.

4. Use according to one of the preceding claims wherein 15% by weight to 50% by weight of monohydric alcohol and/or polyhydric alcohol is present.

5. Use according to one of the preceding claims wherein the alcohol is liquid.

6. Use according to one of the preceding claims wherein the alcohol is 1,4-butanediol, 1,2-propanediol, 1,3-propanediol, 1,3-butanediol, 1,6-hexanediol or a mixture of two or more of these diols.

7. Use according to one of the preceding claims wherein the composition also contains water.

8. Use according to Claim 7 wherein 1% by weight to 10% by weight of water is present.

9. Use according to one of the preceding claims wherein the composition also contains at least one constituent from the group comprising mineral oil, a fatty acid, a fatty acid ester, a compound of formula (1): 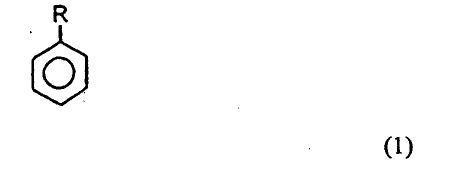 and a compound of formula (2): 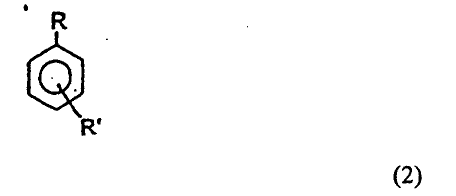 in which R and R' independently of one another are a substituted or unsubstituted alkyl, alkenyl, alkynyl, alkoxy, alkenyloxy or alkynyloxy radical having 1 - 18 carbon atoms, or O-[CH₂-CHR'' -O-]ₘH, R" being H or CH₃ and m being 0 to 13, especially 1 - 4.

10. Use according to Claim 9 wherein the fatty acid is a C₁₀-C₂₄ fatty acid.

11. Use according to Claim 9 wherein the fatty acid ester is an ester of a C₂-C₂₄ fatty acid with a C₁-C₁₅ alcohol.

12. Use according to Claim 9 wherein the fatty acid ester is a C₁₂-C₂₄ plant fatty acid with 1 to 2 units derived from alkylene oxide.

13. Use according to one of Claims 9 to 12 wherein the mineral oil, the fatty acid, the fatty acid ester, the compound of formula (1) and/or the compound of formula (2) make up the remainder of the composition.

## Revendications

1. Utilisation d'une composition, comprenant:
(a) plus de 35% en poids jusqu'à 90% en poids d'un sel d'alcalino-terreux d'un acide alkylbenzènesulfonique, et
(b) 10% en poids à 65% en poids d'alcools mono- et/ou polyvalents, pour la préparation de mousses battues en PVC.

2. Utilisation selon la revendication 1, où 38% en poids à 65% en poids, en particulier 40% en poids à 60% en poids de sel d'alcalino-terreux d'un acide alkylbenzènesulfonique sont présents.

3. Utilisation selon la revendication 1 ou 2, où le sel d'alcalino-terreux d'un acide alkylbenzènesulfonique est le dodécylbenzènesulfonate de calcium.

4. Utilisation selon l'une quelconque des revendications précédentes, où 15% en poids à 50% en poids d'alcools mono- et/ou polyvalents sont présents.

5. Utilisation selon l'une quelconque des revendications précédentes, où l'alcool est liquide.

6. Utilisation selon l'une quelconque des revendications précédentes, où l'alcool est le 1,4-butanediol, le 1,2-propylèneglycol, le 1,3-propanediol, le 1,3-butanedediol, le 1,6-hexanediol ou un mélange de deux ou plusieurs de ceux-ci.

7. Utilisation selon l'une quelconque des revendications précédentes, où la composition contient en outre, de l'eau.

8. Utilisation selon la revendication 7, où 1% en poids à 10% en poids d'eau sont présents.

9. Utilisation selon l'une quelconque des revendications précédentes, où la composition présente d'autre part, au moins un constituant du groupe comportant une huile minérale, un acide gras, un ester d'acide gras, d'un composé de la formule (1): 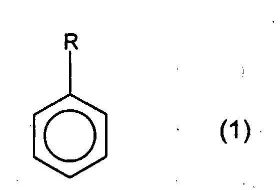 et un composé de la formule (2) : 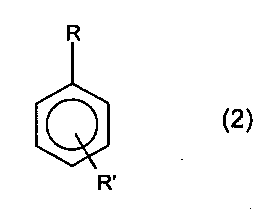 où R et R' représentent indépendamment l'un de l'autre, un reste alkyl, alcényl, alcynyl, alkyloxy, alcényloxy ou alcynyloxy, substitué ou non substitué, avec 1-18 atomes de carbone ou O-[CH₂-CHR"-O-]mH, où R" représente H ou CH₃ et m est 0 à 13, en particulier 1 à 4.

10. Utilisation selon la revendication 9, où l'acide gras est un acide gras en C₁₀-C₂₄.

11. Utilisation selon la revendication 9, où l'ester d'acide gras est un ester d'un acide gras en C₂-C₂₄ avec un alcool en C₁-C₁₅.

12. Utilisation selon la revendication 9, où l'ester d'acide gras est un acide gras végétal en C₁₂-C₂₄ avec 1 ou 2 unités dérivées d'un oxyde d'alcylène.

13. Utilisation selon l'une quelconque des revendications 9 à 12, où l'huile minérale, l'acide gras, l'ester d'acide gras, le composé de formule (1) et/ou le composé de formule (2) forment le reste de la composition.
